# EUROPÄISCHE PATENTANMELDUNG

(11) **EP 4 451 034 A1**
(43) Veröffentlichungstag der Anmeldung: **23.10.2024**
(21) Anmeldenummer: 24170117.6
(22) Anmeldetag: 12.04.2024
(51) Int. Cl.: G02B 19/00, A61B 90/30, A61B 90/50, F21V 5/00

(54) **MEDIZINISCHE LEUCHTE**

(30) Priorität: 20.04.2023 DE 102023110090
(71) Anmelder: Heine Optotechnik GmbH & Co KG, 82205 Gilching (DE)
(72) Erfinder: Michel, Felix, 82205 Gilching (DE); Jetter, Rainer, 82205 Gilching (DE); Raab, Roman, 82205 Gilching (DE)
(74) Vertreter: Flach Bauer & Partner Patentanwälte mbB

(57) **Zusammenfassung**

Eine medizinische Leuchte (10) zur Erzeugung eines Leuchtfelds (F) in einer Leuchtfeldebene (E) hat ein Gehäuse (14) sowie eine Lichtquelle (22) und eine Optik (20), die im Gehäuse (14) angeordnet sind. Die Optik (20) weist einen Kondensor (26) und ein Objektivteil (28) auf, wobei der Kondensor (26) benachbart zur Lichtquelle (22) angeordnet ist und zwei Linsen (32, 34) aufweist, die ein Zwischenbild (Z) des Fernfeldes der Lichtquelle (22) erzeugen. Das Objektivteil (28) bildet das Zwischenbild (Z) des Fernfeldes in die Leuchtfeldebene (E) ab.

## Beschreibung

Die Erfindung betrifft eine medizinische Leuchte zur Erzeugung eines Leuchtfeldes.

Medizinische Leuchten sind bekannt und dienen dazu, ein Leuchtfeld zu erzeugen, um ein Untersuchungs-, Behandlungs- oder Operationsfeld optimal auszuleuchten.

Eine optimale Ausleuchtung besteht darin, dass das Leuchtfeld eine hohe Lichtintensität hat und gleichzeitig eine homogene Beleuchtungsstärkeverteilung aufweist, damit innerhalb des Untersuchungs-, Behandlungs- oder Operationsfelds keine Helligkeitsunterschiede auftreten, die eine Untersuchung, Behandlung oder Operation erschweren würden.

Gleichzeitig sollen medizinische Leuchten möglichst kompakt sein, damit sie mobil sind. Insbesondere soll medizinische Leuchten mittels eines Kopfbands über einen längeren Zeitraum, beispielsweise eine Operation, bequem am Kopf tragbar sein.

Diese Anforderungen sind jedoch gegenläufig, da zur homogenen und hellen Ausleuchtung des Leuchtfelds eine aufwändige Optik notwendig ist, die zu großen Leuchten führt. Kompakte medizinische Leuchten sind jedoch nicht in der Lage, eine homogene Beleuchtungsstärkeverteilung und gleichzeitig ein helles Leuchtfeld zu gewährleisten.

Es ist daher Aufgabe der Erfindung, eine medizinische Leuchte bereitzustellen, die sowohl äußerst kompakt ist als auch ein helles Leuchtfeld mit gleichzeitig homogener Lichtverteilung bereitstellt.

Die Aufgabe wird gelöst durch eine medizinische Leuchte zur Erzeugung eines Leuchtfelds in einer Leuchtfeldebene mit einem Gehäuse sowie einer Lichtquelle und einer Optik, die im Gehäuse angeordnet sind. Die Optik weist einen Kondensor und ein Objektivteil, wobei der Kondensor benachbart zur Lichtquelle angeordnet ist und zwei Linsen aufweist, die ein Zwischenbild des Fernfeldes der Lichtquelle erzeugen. Das Objektivteil bildet das Zwischenbild des Fernfeldes in die Leuchtfeldebene ab.

Zu bemerken ist, dass insbesondere nicht die Lichtquelle und ihre Struktur abgebildet wird, sondern das Fernfeld der Lichtquelle.

Durch die Abbildung des Fernfeldes in ein Zwischenbild durch den Kondensor erhält das Objektivteil, welches das Zwischenbild abbildet, bereits ein Zwischenbild mit homogener Beleuchtungsstärkeverteilung. Daraus folgt, dass auch das vom Objektivteil erzeugte Leuchtfeld eine äußerst homogene Beleuchtungsstärkeverteilung aufweist. Gleichzeitig ermöglicht der Aufbau des Kondensors mit zwei Linsen eine äußerst kompakte Form.

Der Kondensor ist insbesondere unmittelbar benachbart zur Lichtquelle, d.h. ohne weitere optische Komponenten zwischen dem Kondensor und der Lichtquelle.

In einem Aspekt ist die Optik eine abbildende Optik und/oder die Abbildung zur Erzeugung des Zwischenbild ist eine aplanatische Abbildung, sodass die Qualität des Zwischenbildes weiter verbessert werden kann.

Durch den Kondensor kann die als telezentrisch strahlend angenommene Lichtquelle nach Unendlich abgebildet werden, wodurch die Beleuchtungsstärkeverteilung besonders gleichmäßig ist.

Um die Baugröße gering zu halten, kann das Zwischenbild innerhalb des Gehäuses liegen.

In einer Ausführungsform hat das Zwischenbild einen Abstand von der Lichtquelle von weniger als 15 mm, insbesondere weniger als 8 mm, und/oder einen Abstand von der letzten der Linsen des Kondensors von weniger als 4 mm, insbesondere weniger als 3 mm, wodurch eine besonders kompakte Leuchte erreicht wird.

Beispielsweise hat der Kondensor eine Mittendicke von weniger als 15 mm, insbesondere weniger als 5 mm in Richtung der optischen Achse.

In einer Ausgestaltung hat der Kondensor eine numerische Apertur von wenigstens 0,80 insbesondere wenigstens oder gleich 0,85, wodurch die Helligkeit des Leuchtfelds weiter vergrößert wird.

Für eine hochqualitative Abbildung kann die wenigstens eine der Linsen des Kondensors, insbesondere die erste Linse des Kondensors wenigstens eine Fläche haben, die eine asphärische Form hat, insbesondere wobei beide Flächen der jeweiligen Linse eine asphärische Form haben.

Als "Fläche" werden hierbei sowohl die Eintritts- als auch die Austrittsfläche einer Linse bezeichnet.

Beispielsweise hat der Kondensor eine asphärische Sammellinse und eine sphärische Sammellinse.

In einer Ausgestaltung hat die Leuchtfeldebene einen festen Abstand von der Lichtquelle, insbesondere von mehr als 20 cm und/oder weniger als 70 cm, beispielsweise zwischen 30 cm und 40 cm. Insbesondere wenn die Leuchte als Kopfleuchte verwendet wird, wird das Leuchtfeld auf diese Weise in einem für den Träger bzw. dessen Augen angenehmen Abstand erzeugt.

Das Leuchtfeld liegt somit außerhalb des Gehäuses und außerhalb der Leuchte selbst.

Um weiter Platz zu sparen, kann die Optik frei von einer mechanischen Blende, insbesondere einer Lochblende oder einer Irisblende sein.

In einer Ausgestaltung besteht das Objektivteil aus einer oder zwei im Gehäuse fixierten Linsen und/oder hat eine feste Brennweite, wodurch die Komplexität des Objektivteils verringert wird.

In einer Ausführungsform der Erfindung ist das Objektivteil ein pankratisches System mit wenigstens einer verschiebbaren Linsengruppe, die derart im Gehäuse angeordnet ist, dass sie entlang der optischen Achse zwischen zwei Endpositionen verschiebbar ist. Auf diese Weise kann die Größe des Leuchtfeldes ohne Veränderung der numerischen Apertur und daher ohne Veränderung des Lichtstroms angepasst werden. Gegenüber bekannten Systemen, die eine im Durchmesser verstellbare Irisblende zur Veränderung der Leuchtfeldgröße verwenden und die die ausgeleuchtete Blende und nicht das Fernfeld der Lichtquelle im Leuchtfeld abbilden, hat diese erfindungsgemäße Ausführungsform den Vorteil, dass die Beleuchtungsstärke um ein Vielfaches höher ist, zumindest bei kleinen Leuchtfeldgrößen.

Eine Linsengruppe im Sinne dieser Offenbarung kann aus einer, zwei oder mehr als zwei Linsen bestehen.

Die Linsengruppen sind innerhalb des Gehäuses verschiebbar.

Beispielsweise ist das Objektivteil derart ausgebildet, dass es für verschiedenen Positionen der wenigstens einen verschiebbaren Linsengruppe das Zwischenbild des Fernfeldes zu wenigstens 90%, insbesondere vollständig in die Leuchtfeldebene abbildet, insbesondere für die Endpositionen der wenigstens einen Linsengruppe, wodurch eine stufenlose Anpassung des Leuchtfelds ohne Qualitätseinbußen erreicht ist.

Denkbar ist dabei, dass eine der Linsengruppe in das Zwischenbild verfahrbar ist. Im Rahmen dieser Offenbarung wird auch in diesem Falle zur Vereinfachung davon gesprochen, dass das Zwischenbild abgebildet wird, da es in anderen Positionen der Linsengruppe vorhanden ist.

In einer Ausgestaltung sind zwei verschiebbare Linsengruppen vorgesehen, um die Baugröße zu reduzieren.

In einer Ausführungsform weist die Leuchte eine Halterung auf, die das Gehäuse hält, insbesondere wobei die Halterung ein Kopfband zum Tragen auf einem menschlichen Kopf, eine Wandhalterung, eine Tischhalterung, ein Stativ, insbesondere ein Rollstativ, und/oder einen Standfuß aufweist. Auf diese Weise lässt sich die Leuchte sicher an verschiedenen Gegenständen befestigen.

Beispielsweise ermöglicht die Halterung eine Bewegung des Gehäuses gegenüber der übrigen Halterung, insbesondere durch einen flexiblen Arm.

Die Halterung ist insbesondere frei von optischen Komponenten.

In einer Ausgestaltung umfasst die Lichtquelle eine oder mehrere LED, insbesondere wobei die Lichtquelle aus genau einer LED besteht, wodurch die Baugröße weiter reduziert wird.

Beispielsweise sind die eine oder mehrere LEDs frei von einer Linse und/oder frei von Bonddrähten, und/oder die eine oder die mehreren LEDs sind CSP-LEDs (Chip-Scale-Package-LED) oder Flip-Chip LEDs. Mittels solcher LEDs werden Lichtquellen mit sehr hohen Leuchtdichten bei kleiner Baugröße erreicht.

Weitere Merkmale und Vorteile der Erfindung ergeben sich aus der nachfolgenden Beschreibung sowie aus den beigefügten Zeichnungen, auf die Bezug genommen wird. In den Zeichnungen zeigen:
- Fig. 1: eine perspektivische Ansicht einer medizinischen Leuchte gemäß einer Ausführungsform der Erfindung,
- Fig. 2: eine schematische Schnittansicht durch das Gehäuse der Leuchte gemäß Figur 1,
- Fig. 3: eine vergrößerte schematische Ansicht des Kondensors der Leuchte gemäß Figur 1,
- Fig. 4, 5, 6: weitere Ausführungsformen einer medizinischen Leuchte gemäß der Erfindung in schematischen Querschnitten durch das Gehäuse, und
- Fig. 7, 8: weitere Ausführungsformen einer medizinischen Leuchte gemäß der Erfindung in perspektivischen Ansichten.

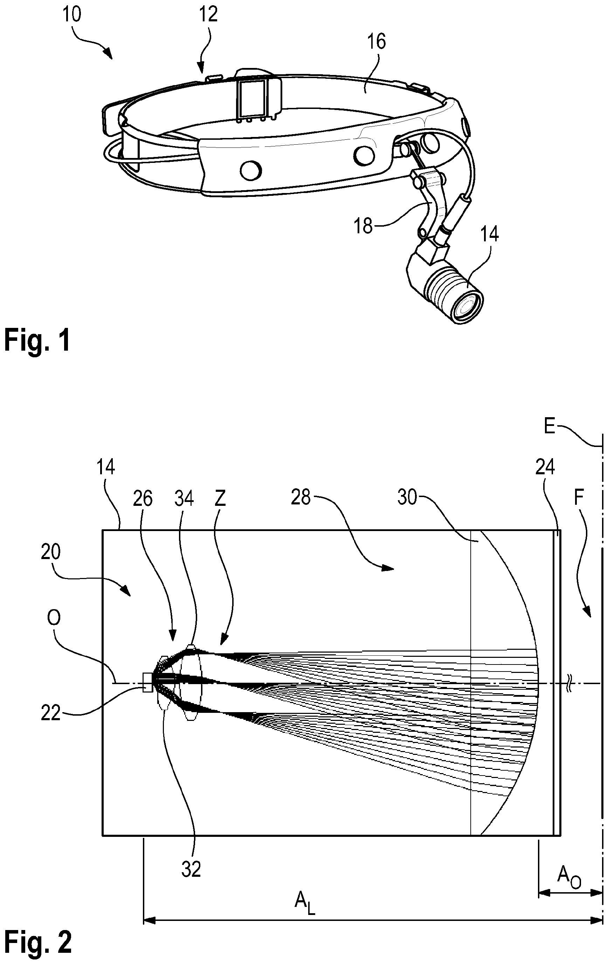

Figur 1 zeigt eine medizinische Leuchte 10 mit einer Halterung 12 und einem Gehäuse 14.

Die Leuchte 10 des ersten Ausführungsbeispiels ist als Kopfleuchte ausgebildet. Die Halterung 12 weist dementsprechend ein Kopfband 16 zum Tragen am Kopf sowie einen Arm 18 auf, der sich vom Kopfband erstreckt und das Gehäuse 14 hält.

Der Arm 18 kann flexibel ausgebildet sein und/oder Gelenke aufweisen. Durch den Arm 18 ist es möglich, das Gehäuse 14 gegenüber der übrigen Halterung, insbesondere dem Kopfband 16 zu bewegen.

Die Halterung 12 ist frei von optischen Komponenten.

Die Leuchte 10 hat zudem eine Stromversorgung, beispielsweise eine Batterie oder ein Akkumulator, die in Figur 1 nicht dargestellt ist.

Figur 2 zeigt einen äußerst schematischen Querschnitt durch das Gehäuse 14.

Innerhalb des Gehäuses 14 ist eine Optik 20 der Leuchte 10 sowie eine Lichtquelle 22 der Leuchte 10 angeordnet.

Das Gehäuse 14 hat einen zylindrischen Innenraum, insbesondere einen kreiszylindrischen Innenraum, in dem die Optik 20 und Lichtquelle 22 angeordnet sind.

An einer ersten Stirnseite hat das Gehäuse 14 eine Wand, wobei die gegenüberliegende, zweite Stirnseite offen ist.

Das Gehäuse 14 hat eine Länge von Stirnseite zu Stirnseite zwischen 40 mm und 100 mm.

Die zweite Stirnseite des Gehäuses 14 ist durch ein Deckglas 24 verschlossen, sodass der Innenraum des Gehäuses 14 verschlossen ist, mit Öffnungen für eine Stromversorgung.

Das Deckglas 24 ist beispielsweise eine planparallele Scheibe die keinen oder nur einen äußerst geringen Einfluss auf den Strahlengang der Optik 20 hat und nicht als Teil der Optik 20 betrachtet wird. Das Deckglas 24 verhindert, dass Schmutz oder Flüssigkeit in das Gehäuse 14 eindringen und die Optik 20 verschmutzen kann.

Die Lichtquelle 22 ist an der ersten Stirnseite des Gehäuses 14 vorgesehen und die Optik 20 ist zwischen der Lichtquelle 22 und dem Deckglas 24 angeordnet. Das Deckglas 24 ist daher auf der von der Lichtquelle 22 abgewandten Seite der Optik 20 angeordnet.

Die Lichtquelle 22 wird als telezentrischer Strahler angenommen. Sie besteht im gezeigten Ausführungsbeispiel aus genau einer LED. Denkbar ist jedoch auch, dass die Lichtquelle 22 eine oder mehrere LED umfasst.

Die LED hat zum Beispiel eine Größe von 1,2 mm x 1,2 mm.

Beispielsweise ist die LED frei von einer Linse und/oder frei von Bonddrähten. Die LED ist als Flip-Chip LED ausgeführt. Denkbar ist auch, dass eine CSP-LED (Chip-Scale-Package-LED) als Lichtquelle 22 verwendet wird.

Die Optik 20 ist eine abbildende Optik und dient dazu, aus dem von der Lichtquelle 22 emittierten Licht ein Leuchtfeld F in einer Leuchtfeldebene E zu erzeugen.

Die Leuchtfeldebene E hat einen festen Abstand A_{L} von der Lichtquelle 22, d.h. von der dem Leuchtfeld F zugewandten Kante der Lichtquelle 22, und einen festen Abstand Ao von der Optik 20, genauer gesagt der letzten Linse des Objektivteils. Die Abstände A_{L} und Ao sind größer als 20 cm und/oder kleiner als 70 cm und liegen beispielsweise zwischen 30 cm und 40 cm.

Das Leuchtfeld F ist daher außerhalb des Gehäuses 14.

Die Optik 20 weist einen Kondensor 26 und ein Objektivteil 28 auf. Insbesondere besteht die Optik 20 aus dem Kondensor 26 und dem Objektivteil 28.

Die Optik 20 hat eine optische Achse O, die der axialen Richtung des Gehäuses 14 entspricht.

In Figur 1 ist der Strahlengang der Lichtstrahlen der Lichtquelle 22 eingezeichnet, die aus dem Teil der Lichtquelle 22 verlaufen, der sich oberhalb der optischen Achse O befindet.

In den Figuren ist der Strahlengang durch den Kondensor 26 und das Objektivteil 28 eingezeichnet.

Im gezeigten Ausführungsbeispiel ist weist das Objektivteil 28 lediglich eine Linse auf, nämlich eine Objektivlinse 30, die im Gehäuse 14 fixiert ist. Insbesondere ist die Objektivlinse 30 nicht entlang der optischen Achse O innerhalb des Gehäuses 14 verschiebbar. Im gezeigten Ausführungsbeispiel ist die Objektivlinse 30 zugleich die letzte Linse des Objektivteils 28.

Die Objektivlinse 30 und/oder andere Linsen des Objektivteils 28 können als achromatische Linsen ausgebildet sein, um Farbfehler am Rand des Leuchtfelds zu reduzieren.

Der Kondensor 26 ist zwischen der Lichtquelle 22 und dem Objektivteil 28 angeordnet.

Beispielsweise ist der Kondensor 26 benachbarte zur Lichtquelle 22, insbesondere unmittelbar benachbarte ohne weitere optische Komponenten zwischen dem Kondensor 26 und der Lichtquelle 22 vorgesehen.

In Richtung der optischen Achse O hat der Kondensor 26 eine Mittendicke von weniger als 15 mm, insbesondere weniger als 5 mm.

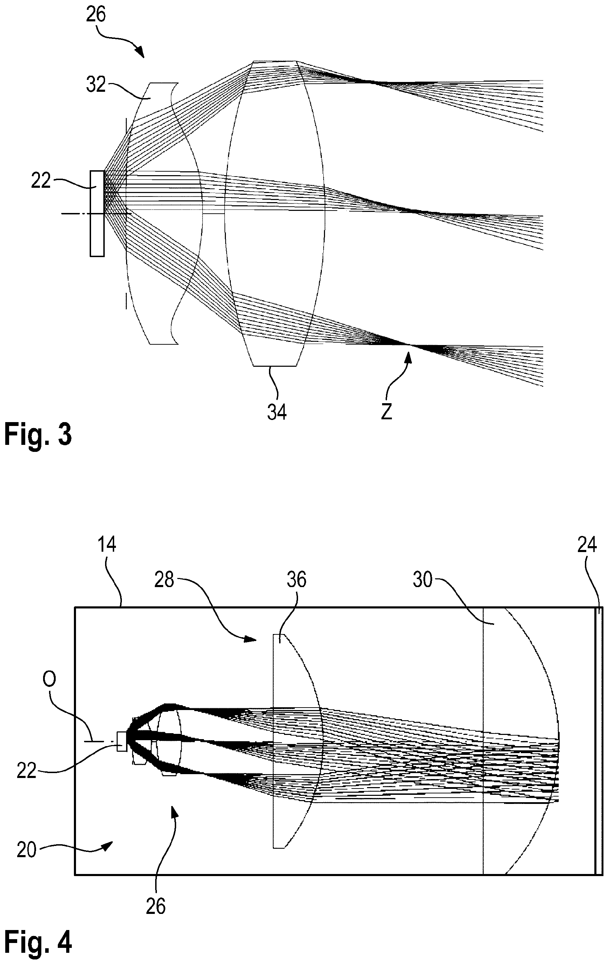

In Figur 3 sind der Kondensor 26 und die Lichtquelle 22 vergrößert dargestellt, wobei auch hier der Strahlengang eingezeichnet ist, der aus der Lichtquelle 22 oberhalb der optischen Achse O austritt.

Der Kondensor 26 hat in der gezeigten Ausführungsform genau zwei Linsen, nämlich eine erste Linse 32 und eine letzte Linse 34, die im gezeigten Ausführungsbeispiel die zweite Linse darstellt.

In anderen Ausführungsformen ist es denkbar, dass der Kondensor 26 mehr als zwei Linsen 32, 34 aufweist.

Der Kondensor 26, und damit die erste Linse 32 und die letzte Linse 34, ist fest im Gehäuse 14 fixiert und nicht entlang der optischen Achse O beweglich.

Der Kondensor 26 hat eine numerische Apertur von wenigstens 0,8, insbesondere von wenigstens oder gleich 0,85.

Die erste Linse 32 ist der Lichtquelle 22 zugewandt und die letzte Linse 34 ist dem Objektivteil 28 zugewandt.

Die Eintritts- und Austrittsflächen der Linsen 32, 34 des Kondensors 26 werden im Rahmen dieser Offenbarung kurz als "Fläche" bezeichnet.

Die Flächen der ersten Linse 32 haben beide eine asphärische Form, die voneinander abweichen.

Denkbar ist auch, dass die Flächen der letzten Linse 34 jeweils eine asphärische Form haben.

Ebenfalls ist es denkbar, dass nur eine Fläche der ersten Linse 32 und/oder nur eine Fläche der letzten Linse 34 asphärische ist.

Beispielsweise hat der Kondensor 26 eine asphärische Sammellinse und eine sphärische Sammellinse als Linsen 32 bzw. 34.

Die erste Linse 32 und die zweite Linse 34 sind derart ausgebildet und angeordnet, dass diese, d. h. der Kondensor 26, ein Zwischenbild Z des Fernfeldes der Lichtquelle 22 erzeugen.

Im Zwischenbild Z des Fernfeldes der Lichtquelle 22 schneiden die Strahlen jeder der Punkte der Lichtquelle 22 einander, wie in Figur 3 gut zu erkennen ist. Dadurch ist die Beleuchtungsstärkeverteilung des Zwischenbildes senkrecht zur optischen Achse O äußerst homogen.

Das Zwischenbild Z des Fernfeldes der Lichtquelle 22 kann auch als Abbildung der Winkelverteilung der Lichtquelle 22 angesehen werden.

Insbesondere ist das Zwischenbild Z keine Abbildung der Lichtquelle 22 und ihrer Struktur. Die Lichtquelle 22 wird nach Unendlich abgebildet.

Das Zwischenbild Z hat zudem einen scharfen Rand senkrecht zur optischen Achse O, d.h. dass die Lichtintensität am Rand nahezu stufenförmig abfällt.

Beispielsweise wird im Rahmen dieser Offenbarung unter einem "scharfen Rand" verstanden, dass der Randbereich des Leuchtfeldes, in dem die Intensität des Leuchtfelds von einer ersten Intensität zu einer zweiten Intensität abnimmt, kleiner als 10% des Radius des Leuchtfelds und/oder kleiner als 2 mm ist (gemessen in der Leuchtfeldebene) ist, insbesondere wobei die erste Intensität zwischen 80% und 95% der maximalen Intensität des Leuchtfelds ist und/oder die zweite Intensität zwischen 10% und 20% der maximalen Intensität des Leuchtfelds ist.

Beispielsweise entspricht der Radius des Leuchtfelds der größten Entfernung der Punkte, an denen das Leuchtfeld die zweite Intensität hat, zum geometrischen Mittelpunkt des Leuchtfelds.

Insbesondere wird unter einem "scharfen Rand" verstanden, dass keine Farbsäume am Rand des Leuchtfelds vorhanden sind. Als Farbsäume können solche Bereiche des Leuchtfelds angesehen werden, in denen die Intensität einzelner Wellenlängenbereiche des sichtbaren Lichts gegenüber anderen Wellenlängenbereichen des sichtbaren Lichts derart stark abgefallen ist, dass dieser Bereich des Leuchtfelds farbig ist.

Eine mechanische Blende, wie eine Lochblende oder eine Irisblende, die einen scharfen Rand des Leuchtfelds erzeugen, sind nicht vorhanden. Der Kondensor 26 und auch die gesamte Optik 20 sind somit frei von einer mechanischen Blende.

Die Linsen 32, 34 erzeugen zudem eine aplanatische Abbildung der Lichtquelle. Die Abbildung zur Erzeugung des Zwischenbild Z genügt damit einer der Aplanasiebedingungen.

Das Zwischenbild Z hat einen Abstand von der Lichtquelle 22 von weniger als 15 mm, insbesondere von weniger als 8 mm.

Der Abstand zwischen der letzten Linse 34 des Kondensors 26 und dem Zwischenbild Z beträgt weniger als 4 mm, insbesondere weniger als 3 mm.

Das Zwischenbild Z liegt somit innerhalb des Gehäuses 14. Im gezeigten Ausführungsbeispiel mit feststehenden Linsen 30 des Objektivteils 28 zwischen dem Kondensor 26 und dem Objektivteil 28.

Das erzeugte Zwischenbild Z wird, wie in Figur 2 zu sehen ist, vom Objektivteil 28, in der ersten Ausführungsform somit von der Linse 30, in die Leuchtfeldebene E abgebildet. Die Abbildung des Zwischenbildes Z in der Leuchtfeldebene E ist das Leuchtfeld F.

Das Leuchtfeld F hat einen scharfen Rand in der Leuchtfeldebene E, da bereits das Zwischenbild Z einen scharfen Rand aufweist. Zugleich ist das Leuchtfeld F äußerst homogen ausgeleuchtet, da das Zwischenbild Z eine äußerst homogene Beleuchtungsstärkeverteilung hat.

Die Qualität des Leuchtfelds F, insbesondere die Homogenität der Beleuchtungsstärkeverteilung und die Definiertheit des Randes des Leuchtfelds F, sind somit maßgeblich abhängig von der Qualität des Zwischenbildes Z.

Die hohe Qualität des Zwischenbildes Z ist dem Kondensor 26 geschuldet, sodass durch den Kondensor 26 ein hochqualitatives Leuchtfeld F mit hoher Intensität und homogene Beleuchtungsstärkeverteilung ermöglicht wird. Zugleich wird eine äußerst kompakte Bauform erreicht.

Die Figuren 4 bis 8 zeigen weitere Ausführungsformen der Erfindung, die im Wesentlichen der ersten Ausführungsform entsprechen. Im Folgenden wird daher lediglich auf die Unterschiede eingegangen und gleiche und funktionsgleiche Teile sind mit denselben Bezugszeichen versehen.

Den Ausführungsformen ist vor allen Dingen gemein, dass sie den gleichen Kondensor 26 und den gleichen Aufbau der Lichtquelle 22 relativ zum Kondensor 26 aufweisen.

In allen Ausführungsformen wird somit das hochqualitative Zwischenbild Z erzeugt.

In Figur 4 ist eine weitere Ausführungsform der medizinischen Leuchte 10 in einer Ansicht entsprechend der Figur 2 dargestellt.

Im Unterschied zur ersten Ausführungsform hat die medizinischen Leuchte 10 der zweiten Ausführungsform ein anderes Objektivteil 28. Das Objektivteil 28 der zweiten Ausführungsform weist zwei Linsen auf, nämlich eine erste Objektivlinse 36 und die Objektivlinse 30 als zweite Linse.

Die erste Objektivlinse 30 ist benachbart zum Kondensor 26 angeordnet und die Objektivlinse 30 ist benachbart zum Deckglas 24 vorgesehen.

Die Linsen 30, 36 des Objektivteils 28 sind wie schon wie zur ersten Ausführungsform diskutiert im Gehäuse 14 fixiert, sodass auch das Objektivteil 28 der zweiten Ausführungsform eine feste Brennweite hat.

Im Unterschied zur ersten Ausführungsform ist der Durchmesser des Leuchtfelds F in der Leuchtfeldebene E größer.

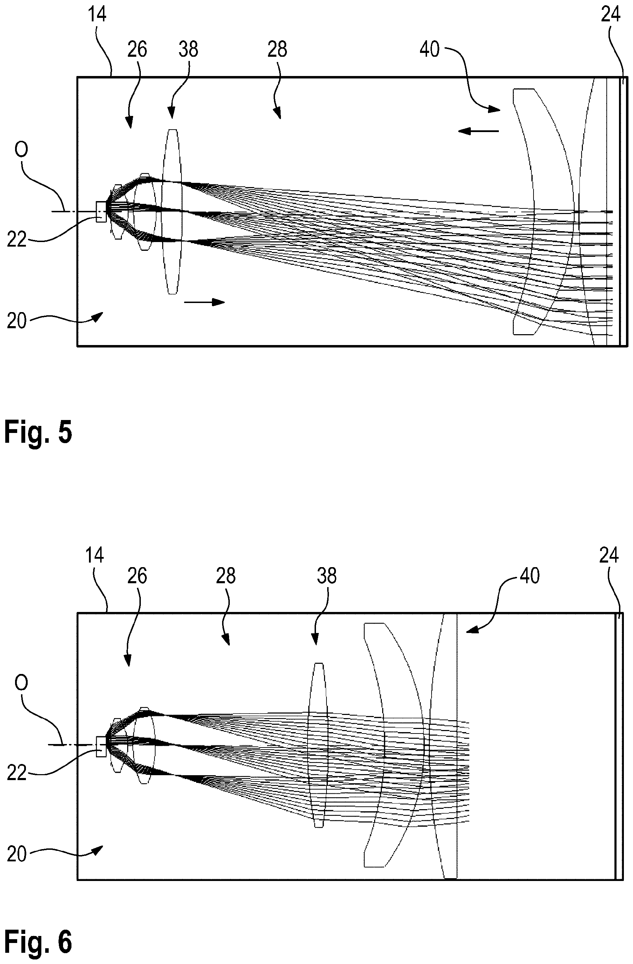

In den Figuren 5 und 6 ist eine dritte Ausführungsform der medizinischen Leuchte 10 in schematischen Darstellungen entsprechend der der Figur 2 dargestellt.

Auch in der dritten Ausführungsform wird ein anderes Objektivteil 28 verwendet.

Das Objektivteil 28 in der dritten Ausführungsform ist ein pankratisches System mit zwei Linsengruppen, nämlich eine ersten Linsengruppe 38 und einer zweiten Linsengruppe 40.

Die Linsengruppen 38, 40 können eine, zwei oder mehr Linsen aufweisen oder daraus bestehen. Im gezeigten Ausführungsbeispiel besteht die erste Linsengruppe 38 aus einer einzelnen Linse und die zweite Linsengruppe 40 besteht aus zwei Linsen.

Die Linsengruppen 38, 40 sind entlang der optischen Achse verschiebbar im Gehäuse 14 gelagert. Beispielsweise sind die Linsengruppen 38, 40 über eine gemeinsame Mechanik verschiebbar, die derart ausgeführt ist, dass die Linsengruppen 38, 40 zwischen zwei Endpositionen verschiebbar sind. Die Figuren 5 und 6 zeigen die Stellung der Linsengruppen 38, 40 in jeweils einer der beiden Endpositionen.

Beim Verschieben zwischen den Endpositionen werden die Linsengruppen 38, 40 überwiegend gegenläufig zueinander verschoben, wie durch die Pfeile in Figur 5 angedeutet.

Das Objektivteil 28 bildet ebenso wie zur ersten Ausführungsform diskutiert das Zwischenbild Z in die Leuchtfeldebene E ab, d.h. zu wenigstens 90 %, insbesondere jedoch vollständig. Dies gilt insbesondere für die Endpositionen, aber auch für jede Stellung der Linsengruppen 38, 40 zwischen den Endpositionen.

In der in Figur 5 gezeigten Endposition ist die erste Linsengruppe 38 so nah am Kondensor 26 platziert, dass das Zwischenbild Z innerhalb der ersten Linsengruppe 38 bzw. dahinter entstehen würde. Zur Vereinfachung wird im Rahmen dieser Offenbarung jedoch auch davon gesprochen, dass das Zwischenbild abgebildet wird, da es vorhanden ist, wenn sich die Linsengruppen 38, 40 in anderen Positionen befinden. Insbesondere ist das Zwischenbild Z für den überwiegenden Anteil der möglichen Stellungen zwischen den beiden Endpositionen der Linsengruppen 38, 40 vorhanden.

Das Objektivteil 28 der dritten Ausführungsform wirkt ähnlich einem Varioobjektiv für Kameras. Diese Ausführungsform stellt ein pankratisches optisches System dar. Die Größe des Leuchtfelds F in der Leuchtfeldebene E ist von der Position der Linsengruppen 38, 40 abhängig, wobei die in Figur 5 gezeigte Endpositionen das Leuchtfeld F und die in Figur 6 gezeigte Endpositionen das größte Leuchtfeld F in der Leuchtfeldebene E erzeugt.

Bei dem Objektivteil 28 der dritten Ausführungsform ist nicht nur die Brennweite verstellbar, sondern es bleibt auch die Bild- und Objektweite gleich. Dies bedeutet, dass der Rand des Leuchtfelds F bei Verstellung der Größe des Leuchtfelds stets scharf bleibt.

Auch bei dieser Abbildung handelt es sich um eine vollständige optische Abbildung, sodass aufgrund der Entendue-Erhaltung nicht nur das Leuchtfeld F vergrößert oder verkleinert wird, sondern gleichzeitig auch die Intensität innerhalb des Leuchtfelds F. Beispielsweise wird die Intensität erhöht, wenn das Leuchtfeld F verkleinert wird, da zu jedem Zeitpunkt nahezu die gesamte von der Lichtquelle 22 emittierte Lichtleistung in das Leuchtfeld F abgebildet wird.

Dies ist ein wesentlicher Unterschied zu solchen medizinischen Leuchten 10 aus dem Stand der Technik, in denen das Leuchtfeld mittels einer mechanischen Blende verkleinert werden kann. In diesem Falle verändert sich die Intensität des Leuchtfelds F und das Licht geht an der mechanischen Blende verloren.

Mit der erfindungsgemäßen Leuchte 10 wird somit eine kompakte medizinische Leuchte 10 bereitgestellt, die ein Leuchtfeld F mit variabler Größe und variabler Intensität bereitstellt. Gleichzeitig ist die medizinische Leuchte 10 auch in der dritten Ausführungsform derart kompakt, dass sie mittels des Kopfbandes 16 am Kopf getragen werden kann.

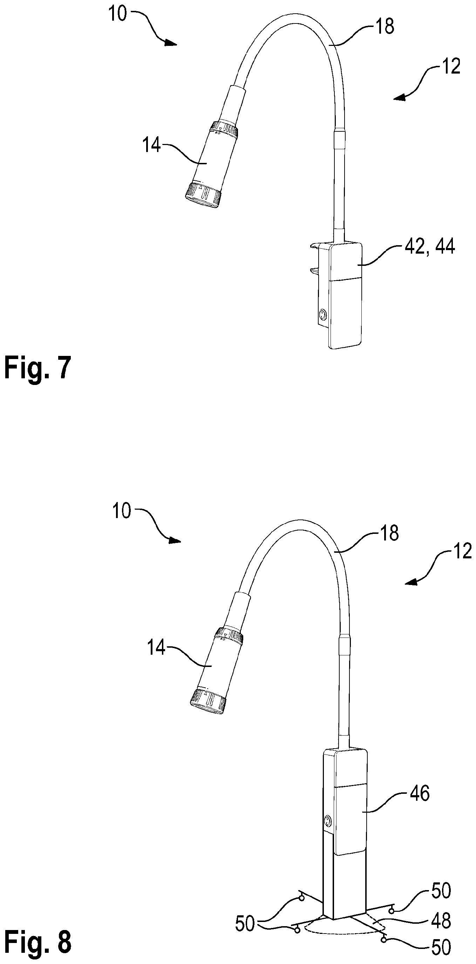

In Figur 7 ist eine weitere Ausführungsform der medizinischen Leuchte 10 dargestellt, die sich durch ihre Halterung 12 von den ersten drei Ausführungsformen unterscheidet.

In der in Figur 7 gezeigten weiteren Ausführungsform umfasst die Halterung 12 eine Wandhalterung 42 oder eine Tischhalterung 44 zur Befestigung der medizinischen Leuchte 10 an einer Tischplatte.

Das Gehäuse 14 ist über den Arm 18 gegenüber der Wandhalterung 42 bzw. der Tischhalterung 44 beweglich.

In Figur 8 ist eine weitere Ausführungsform der medizinischen Leuchte 10 mit einer weiteren alternativen Halterung 12 dargestellt.

Die Halterung 12 der Ausführungsform gemäß Figur 8 weist ein Stativ 46 oder einen Standfuß 48 (gestrichelt dargestellt) auf. Insbesondere kann das Stativ 46 mehrere Rollen 50 aufweisen, also ein Rollstativ sein.

Die Merkmale der verschiedenen Ausführungsformen können beliebig miteinander kombiniert werden. Insbesondere können die verschiedenen Ausführungen der Objektivteile 28 beliebig mit den Ausführungen der Halterung 12 kombiniert werden.

## Patentansprüche

1. Medizinische Leuchte zur Erzeugung eines Leuchtfelds (F) in einer Leuchtfeldebene (E) mit einem Gehäuse (14) sowie einer Lichtquelle (22) und einer Optik (20), die im Gehäuse (14) angeordnet sind,
wobei die Optik (20) einen Kondensor (26) und ein Objektivteil (28) aufweist,
wobei der Kondensor (26) benachbart zur Lichtquelle (22) angeordnet ist und zwei Linsen (32, 34) aufweist, die ein Zwischenbild (Z) des Fernfeldes der Lichtquelle (22) erzeugen, und
wobei das Objektivteil (28) das Zwischenbild (Z) des Fernfeldes in die Leuchtfeldebene (E) abbildet.

2. Leuchte nach Anspruch 1, **dadurch gekennzeichnet, dass** die Optik (20) eine abbildende Optik ist und/oder die Abbildung zur Erzeugung des Zwischenbilds (Z) eine aplanatische Abbildung ist.

3. Leuchte nach einem der vorhergehenden Ansprüche, **dadurch gekennzeichnet, dass** das Zwischenbild (Z) innerhalb des Gehäuses (14) liegt.

4. Leuchte nach einem der vorhergehenden Ansprüche, **dadurch gekennzeichnet, dass** das Zwischenbild (Z) einen Abstand von der Lichtquelle (22) von weniger als 15 mm, insbesondere weniger als 8 mm, und/oder einen Abstand von der letzten der Linsen (34) des Kondensors (26) von weniger als 4 mm, insbesondere weniger als 3 mm hat.

5. Leuchte nach einem der vorhergehenden Ansprüche, **dadurch gekennzeichnet, dass** der Kondensor (26) eine numerische Apertur von wenigstens 0,80 insbesondere wenigstens oder gleich 0,85 hat.

6. Leuchte nach einem der vorhergehenden Ansprüche, **dadurch gekennzeichnet, dass** wenigstens eine der Linsen (32, 34) des Kondensors (26), insbesondere die erste Linse (32) des Kondensors (26) wenigstens eine Fläche hat, die eine asphärische Form hat, insbesondere wobei beide Flächen der jeweiligen Linse (32, 34) eine asphärische Form haben.

7. Leuchte nach einem der vorhergehenden Ansprüche, **dadurch gekennzeichnet, dass** die Leuchtfeldebene (E) einen festen Abstand (A_{L}, Ao) von der Lichtquelle (22) hat, insbesondere von mehr als 20 cm und/oder weniger als 70 cm, beispielsweise zwischen 30 cm und 40 cm.

8. Leuchte nach einem der vorhergehenden Ansprüche, **dadurch gekennzeichnet, dass** die Optik (20) frei von einer mechanischen Blende, insbesondere einer Lochblende oder einer Irisblende ist.

9. Leuchte nach einem der vorhergehenden Ansprüche, **dadurch gekennzeichnet, dass** das Objektivteil (28) aus einer oder zwei im Gehäuse (14) fixierten Linsen (32, 34) besteht und/oder eine feste Brennweite hat.

10. Leuchte nach einem der Ansprüche 1 bis 8, **dadurch gekennzeichnet, dass** das Objektivteil (28) ein pankratisches System mit wenigstens einer verschiebbaren Linsengruppe (38, 40) ist, die derart im Gehäuse (14) angeordnet ist, dass sie entlang der optischen Achse (O) zwischen zwei Endpositionen verschiebbar ist.

11. Leuchte nach Anspruch 10, **dadurch gekennzeichnet, dass** das Objektivteil (28) derart ausgebildet ist, dass es für verschiedenen Positionen der wenigstens einen verschiebbaren Linsengruppe (38, 40) das Zwischenbild (Z) des Fernfeldes zu wenigstens 90%, insbesondere vollständig in die Leuchtfeldebene (E) abbildet, insbesondere für die Endpositionen der wenigstens einen Linsengruppe (38, 40).

12. Leuchte nach Anspruch 10 oder 11, **dadurch gekennzeichnet, dass** zwei verschiebbare Linsengruppen (38, 40) vorgesehen sind.

13. Leuchte nach einem der vorhergehenden Ansprüche, **dadurch gekennzeichnet, dass** die Leuchte (10) eine Halterung (12) aufweist, die das Gehäuse (14) hält, insbesondere wobei die Halterung (12) ein Kopfband (16) zum Tragen auf einem menschlichen Kopf, eine Wandhalterung (42), eine Tischhalterung (44), ein Stativ (46), insbesondere ein Rollstativ, und/oder einen Standfuß (48) aufweist.

14. Leuchte nach einem der vorhergehenden Ansprüche, **dadurch gekennzeichnet, dass** die Lichtquelle (22) eine oder mehrere LED umfasst, insbesondere wobei die Lichtquelle (22) aus genau einer LED besteht.

15. Leuchte nach Anspruch 14, **dadurch gekennzeichnet, dass** die eine oder mehrere LEDs frei von einer Linse sind und/oder frei von Bonddrähten sind und/oder dass die eine oder die mehreren LEDs CSP-LEDs oder Flip-Chip LEDs sind.
